(19) **Europäisches Patentamt / European Patent Office / Office européen des brevets**

(11) **EP 3 530 640 B1**

(12) **EUROPEAN PATENT SPECIFICATION**

(45) Date of publication and mention
of the grant of the patent:
**08.03.2023 Bulletin 2023/10**

(51) International Patent Classification (IPC):
**C07C 1/12** *(2006.01)*  **C07C 9/04** *(2006.01)*
**C10L 1/08** *(2006.01)*  **C10L 3/08** *(2006.01)*

(21) Application number: **19157367.4**

(22) Date of filing: **15.02.2019**

(52) Cooperative Patent Classification (CPC):
(C-Sets available)
**C10L 3/08; C07C 1/12;** C10L 2290/02;
C10L 2290/06; C10L 2290/08; C10L 2290/10;
C10L 2290/12; C10L 2290/46; C10L 2290/542;
C10L 2290/58          (Cont.)

(54) **METHANE PRODUCTION DEVICE AND METHANE PRODUCTION METHOD**

VORRICHTUNG ZUR METHANHERSTELLUNG UND VERFAHREN ZUR METHANHERSTELLUNG

DISPOSITIF ET PROCÉDÉ DE PRODUCTION DE MÉTHANE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.02.2018 JP 2018028331**

(43) Date of publication of application:
**28.08.2019 Bulletin 2019/35**

(73) Proprietor: **Kabushiki Kaisha Toyota Chuo
Kenkyusho
Nagakute-shi, Aichi 480-1192 (JP)**

(72) Inventors:
 • **HIROTA, Yasuki
  Nagakute-shi, Aichi 480-1192 (JP)**
 • **YAMAUCHI, Takafumi
  Nagakute-shi, Aichi-ken 480-1192 (JP)**
 • **SHICHI, Akira
  Nagakute-shi, Aichi-ken 480-1192 (JP)**
 • **YAMAMOTO, Seiji
  Nagakute-shi, Aichi-ken 480-1192 (JP)**
 • **KUNITOMI, Seiichi
  Nagakute-shi, Aichi-ken 480-1192 (JP)**
 • **IMAGAWA, Haruo
  Nagakute-shi, Aichi 480-1192 (JP)**
 • **SAYAMA, Shogo
  Nagakute-shi, Aichi 480-1192 (JP)**
 • **OGASAWARA, Kazuto
  Nagakute-shi, Aichi-ken 480-1192 (JP)**
 • **SETOYAMA, Norihiko
  Nagakute-shi, Aichi-ken 480-1192 (JP)**

 • **SAKAI, Masatoshi
  Nagakute-shi, Aichi 480-1192 (JP)**
 • **YAMAZAKI, Kiyoshi
  Nagakute-shi, Aichi-ken 480-1192 (JP)**
 • **BABA, Naoki
  Nagakute-shi, Aichi 480-1192 (JP)**
 • **KUZUYA, Takashi
  Nagakute-shi, Aichi-ken 480-1192 (JP)**
 • **TANAKA, Toshiyuki
  Nagakute-shi, Aichi-ken 480-1192 (JP)**

(74) Representative: **Kramer Barske Schmidtchen
Patentanwälte PartG mbB
European Patent Attorneys
Landsberger Strasse 300
80687 München (DE)**

(56) References cited:
**DE-A1-102013 022 021**

 • **DATABASE WPI Week 201517 Thomson
  Scientific, London, GB; AN 2015-05566W
  XP002792824, & CN 104 152 197 A (CAS DALIAN
  CHEM & PHYSICAL INST) 19 November 2014
  (2014-11-19) -& CN 104 152 197 A (CAS DALIAN
  CHEM & PHYSICAL INST) 19 November 2014
  (2014-11-19)**
 • **GÖKHAN ALPTEKIN ET AL: "An Advanced CO2
  Removal and Reduction System", SAE
  TECHNICAL PAPER SERIES, 19 July 2004
  (2004-07-19), pages 1-7, XP055604258, US ISSN:
  0148-7191, DOI: 10.4271/2004-01-2445**

EP 3 530 640 B1

(52) Cooperative Patent Classification (CPC): (Cont.)

C-Sets
**C07C 1/12, C07C 9/04**

**Description**

The present disclosure relates to a methane production device and a methane production method.

Background

A conventionally known technique of producing methane $CH_4$ recycles carbon dioxide $CO_2$ from an exhaust gas discharged from a combustion furnace or the like and causes the carbon dioxide $CO_2$ to react with hydrogen $H_2$ (as described in, for example, WO 2016/076041A, WO 2011/108546A, JP 2009-269983A, JP 5959036B, JP H11-46460A, and JP 2009-77457A). For example, WO 2016/076041A discloses a technique of converting the waste heat of an internal combustion engine into electricity and producing $H_2$ by using the electricity in a $CO_2$ recycle device configured to generate methane from $CO_2$ included in an exhaust gas and $H_2$ supplied from a water electrolyzer. WO 2011/108546A discloses a technique of producing $H_2$ by using heat generated by methanation reaction in the process of producing methane from $CO_2$ and $H_2$. JP 2009-269983A discloses a technique of obtaining methane by using the pressure and the heat supplied from an internal combustion engine to a reaction chamber where an exhaust gas discharged from the internal combustion engine is mixed with $H_2$ supplied from a water electrolyzer. DE 10 2013 022 021 A discloses a method for methanization of $CO_2$ obtained from gas mixtures after separation by selective reversible adsorption. CN 104152197 A discloses a process and a device for methanization of $CO_2$ obtained from the atmosphere in closed systems such as space vehicles. Gökhab Alpetkin et al.: "An Advanced CO2 Removal and Reduction System", SAE TECHNICAL PAPER SERIES 2004-01-2445, 2004, ISSN 0148-7191, discloses another system for removing $CO_2$ from the atmosphere in a space vehicle.

Regardless of the above prior arts, however, there is still room for improvement in the technique of enhancing the system efficiency of the methane production device. The system efficiency herein denotes an index relating to the energy efficiency in production of methane. The system efficiency is proportional to a conversion ratio and decreases with an increase in externally applied energy (external power). The technique described in WO 2016/076041A converts the waste heat into electricity and uses the electricity for production of H2 to recycle the thermal energy. A loss is, however, likely to occur in the course of conversion. There is accordingly still room for improvement in enhancing the system efficiency. The technique described in WO 2011/108546A recycles the thermal energy but uses the heat of the methanation reaction for production of H2. This technique is not sufficient from the point of view in enhancing the system efficiency. The technique described in JP 2009-269983A uses the waste heat of the engine to form a reaction field of the methanation reaction that is an exothermic reaction and accordingly does not provide a sufficiently high system efficiency. The technique described in JP 2009-269983A causes the exhaust gas to direction react with $H_2$ and is thus likely to reduce the reaction efficiency and the quality of the generated gas.

Summary

In order to solve the problem described above, one object of the present disclosure is to provide a technique that enhances the system efficiency of a methane production device configured to produce methane from $H_2$ and $CO_2$.

At least part of the problem described above is solved by a methane production device according to claim 1 or a methane production method according to claim 6.

The methane production device injects hydrogen to the inside of the separator. This decreases the CO2 partial pressure in the gas phase and enables carbon dioxide to be taken out from inside of the separator. This configuration reduces the external power of a vacuum pump or the like to take out carbon dioxide. This configuration accordingly enhances the system efficiency of the methane production device.

The methane production device of claim 2 enables the heat of the methanation reaction generated in the reactor to be supplied to the separator and thereby uses the thermal energy generated by the methanation reaction as thermal energy required to desorb carbon dioxide from the adsorbent. This configuration accordingly reduces the external power to supply thermal energy to the separator. This configuration thus further enhances the system efficiency of the methane production device.

The configuration of claim 3 enables a larger amount of heat to be supplied to the downstream side that is farther from the hydrogen injector and is likely to adsorb carbon dioxide again. This configuration thus enhances the recycle rate of carbon dioxide from the separator.

The methane production device of claim 4 enables the heat of the methanation reaction generated in the reactor to be supplied to the separator by using the hydrogen. There is accordingly no need to provide a circulation device of oil or the like. This achieves downsizing of the methane production device. This configuration also enables the heated hydrogen gas to be injected and thereby more efficiently supplies the thermal energy to the adsorbent.

The methane production device of claim 5 enables the heat of the methanation reaction generated in the reactor to be supplied to the separator by the simple configuration. This accordingly achieves downsizing of the methane production device.

The methane production device allows for adjustment of the ratio of hydrogen and carbon dioxide supplied to the reactor and thereby further enhances the conversion ratio.

The methane production device enables the ratio of hydrogen and carbon dioxide included in the material gas that is supplied to the reactor to be close to a target value such as a stoichiometric ratio of the methanation reaction and thereby further enhances the conversion ratio.

Brief Description of Drawings

Fig. 1 is a diagram illustrating the schematic configuration of a methane production device according to a first embodiment;
Fig. 2 is a flowchart showing a flow path control process;
Fig. 3 is a diagram illustrating the flow state of gas and the like at steps S13 to S15;
Fig. 4 is a diagram illustrating the flow state of gas and the like at steps S23 to S25;
Fig. 5 is a diagram illustrating the schematic configuration of a methane production device according to Comparative Example 1;
Fig. 6 is a graph showing comparison of $CO_2$ recycle energy between the embodiment and Comparative Examples 1 to 3;
Fig. 7 is a graph showing comparison of system efficiency between the embodiment and Comparative Example 1;
Fig. 8 is a diagram illustrating the internal configuration of a $CO_2$ separator;
Fig. 9 is a diagram illustrating the schematic configuration of a methane production device according to a second embodiment;
Fig. 10 is a diagram illustrating the schematic configuration of a methane production device according to a third embodiment; and
Fig. 11 is a diagram illustrating the schematic configuration of a methane production device according to a fourth embodiment.

Description of Embodiments

First Embodiment

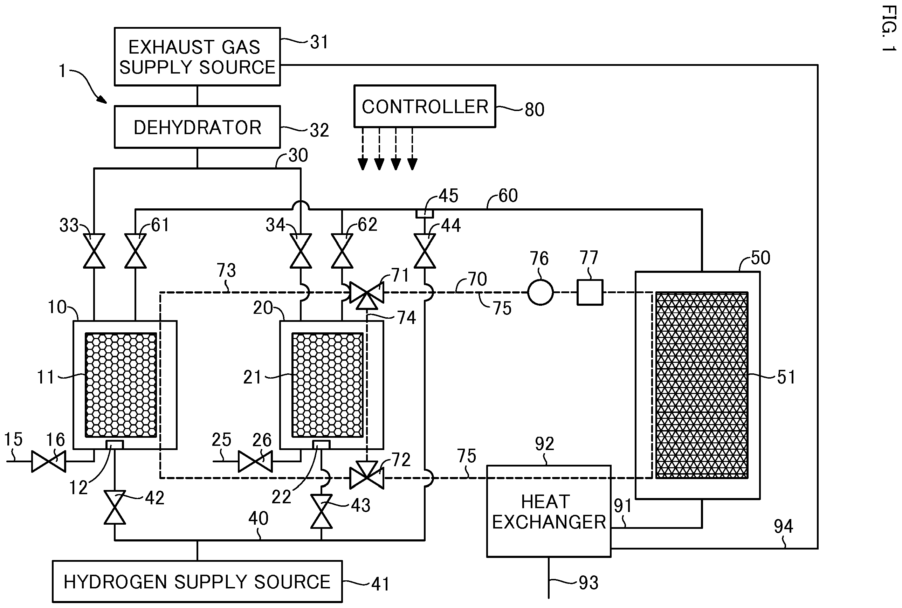

Fig. 1 is a diagram illustrating the schematic configuration of a methane production device 1 according to a first embodiment. The methane production device 1 includes a first $CO_2$ separator 10, a second $CO_2$ separator 20, an exhaust gas supply flow path 30, a hydrogen supply flow path 40, a reactor 50, a material gas flow path 60, a heat medium flow path 70 and a controller 80.

The first $CO_2$ separator 10 is a device configured to separate and recycle $CO_2$ from the exhaust gas and includes an adsorbent 11 placed inside thereof. The adsorbent 11 is a material having $CO_2$ storage (adsorption) capacity and is, for example, zeolite, active carbon or silica gel. The first $CO_2$ separator 10 also includes a hydrogen injector 12 provided inside of the first $CO_2$ separator 10 to inject hydrogen that is supplied through the hydrogen supply flow path 40, to the inside of the first $CO_2$ separator 10. The first $CO_2$ separator 10 is connected with the exhaust gas supply flow path 30, the material gas flow path 60 and a first discharge flow path 15. While $CO_2$ included in the exhaust gas supplied from the exhaust gas supply flow path 30 is stored in the adsorbent 11, the remaining components of the exhaust gas are discharged from the first discharge flow path 15 via a first discharge valve 16 to the outside. $CO_2$ desorbed from the adsorbent 11 is purged by $H_2$ injected from the hydrogen injector 12 and is delivered along with $H_2$ to the material gas flow path 60.

The second $CO_2$ separator 20 is a device having the identical configuration and the identical capacity to those of the first $CO_2$ separator 10 and includes an adsorbent 21 placed inside thereof. Like the adsorbent 11, the adsorbent 21 is a material that is capable of storing $CO_2$ and has an equivalent storage capacity to that of the adsorbent 11. The second $CO_2$ separator 20 also includes a hydrogen injector 22 provided inside of the second $CO_2$ separator 20 to inject hydrogen that is supplied through the hydrogen supply flow path 40, to the inside of the second $CO_2$ separator 20. The second $CO_2$ separator 20 is connected with the exhaust gas supply flow path 30, the material gas flow path 60 and a second discharge flow path 25. While $CO_2$ included in the exhaust gas supplied through the exhaust gas supply flow path 30 is stored in the adsorbent 21, the remaining components of the exhaust gas are discharged from the second discharge flow path 25 via a second discharge valve 26 to the outside. $CO_2$ desorbed from the adsorbent 21 is purged by $H_2$ injected from the hydrogen injector 22 and is delivered along with $H_2$ to the material gas flow path 60.

The exhaust gas supply flow path 30 is a gas flow path provided to supply the exhaust gas that is discharged from an exhaust gas supply source 31, to the first $CO_2$ separator 10 and the second $CO_2$ separator 20 and is configured to include a plurality of gas pipings. The exhaust gas supply flow path 30 is provided with a dehydrator 32, a first exhaust gas supply valve 33 and a second exhaust gas supply valve 34. The exhaust gas supply source 31 is, for example, a combustion furnace at factory. The exhaust gas includes, for example, $O_2$, $N_2$ and $H_2O$ other than $CO_2$. The exhaust gas discharged from the exhaust gas supply source 31 is dehydrated by the dehydrator 32. The dehydrated exhaust gas is then supplied through the first exhaust gas supply valve 33 to the first $CO_2$ separator 10 and is also supplied through the second exhaust gas supply valve 34 to the second $CO_2$ separator 20. The first exhaust gas supply valve 33 and the second exhaust gas supply valve 34 are respectively controlled to be opened and closed by the controller 80. The exhaust gas supply flow path 30 is provided with a temperature sensor, a flow rate sensor and a $CO_2$ concentration sensor (not shown) configured to measure the temperature, the flow rate and the $CO_2$ concentration of the exhaust gas that is supplied to the first $CO_2$ separator 10 and to the second $CO_2$ separator 20.

The hydrogen supply flow path 40 is a gas flow path provided to supply $H_2$ of a hydrogen supply source 41 to the first $CO_2$ separator 10, the second $CO_2$ separator 20 and the material gas flow path 60 and is configured to include a plurality of gas pipings. The hydrogen supply flow path 40 is provided with a first hydrogen supply valve 42, a second hydrogen supply valve 43 and a third hydrogen supply valve 44. The hydrogen supply source 41 is, for example, a water electrolyzer or a hydrogen tank. The following description is on the assumption that a water electrolyzer is employed as the hydrogen supply source 41. $H_2$ generated by the hydrogen supply source 41 is injected to the inside of the first $CO_2$ separator 10 when the first hydrogen supply valve 42 is opened, while being injected to the inside of the second $CO_2$ separator 20 when the second hydrogen supply valve 43 is opened. Furthermore, $H_2$ is added from a hydrogen adding portion 45 to the material gas flowing through the material gas flow path 60, when the third hydrogen supply valve 44 is opened. The first hydrogen supply valve 42, the second hydrogen supply valve 43 and the third hydrogen supply valve 44 are respectively controlled to be opened and closed. The hydrogen supply flow path 40 is provided with a temperature sensor and a flow rate sensor (not shown) configured to measure the temperature and the flow rate of the $H_2$ gas that is supplied to the first $CO_2$ separator 10, the second $CO_2$ separator 20 and the material gas flow path 60.

The reactor 50 is a vessel configured to generate methane inside thereof by methanation reaction and includes a catalyst 51 placed inside thereof. The catalyst 51 includes a metal having methanation performance. Examples of the metal having the methanation performance include Ru and Ni. The reactor 50 is connected with the material gas flow path 60 and a reactive gaseous mixture flow path 91. The material gas including $CO_2$ and $H_2$ is supplied through the material gas flow path 60 into the reactor 50 and is subjected to the methanation reaction to generate methane. The reactive gaseous mixture including the generated methane is supplied through the reactive gaseous mixture flow path 91 to a heat exchanger 92. The heat exchanger 92 serves to separate $H_2O$ from the reactive gaseous mixture. The separated $H_2O$ is discharged through an $H_2O$ discharge flow path 93 to the outside. The remaining reactive gas after separation of $H_2O$ is supplied through a reactive gas flow path 94 to the exhaust gas supply source 31.

The material gas flow path 60 is a gas flow path provided to supply the material gas that is delivered from the first $CO_2$ separator 10 and the second $CO_2$ separator 20 and includes $H_2$ and $CO_2$, and is configured to include a plurality of gas pipings. The material gas flow path 60 is provided with a first material gas valve 61 and a second material gas valve 62. The first material gas valve 61 and the second material gas valve 62 are controlled to be opened and closed by the controller 80. The controller 80 controls the first material gas valve 61 to be opened and the second material gas valve 62 to be closed, in order to supply the material gas from the first $CO_2$ separator 10 to the reactor 50. The controller 80 controls the first material gas valve 61 to be closed and the second material gas valve 62 to be opened, in order to supply the material gas from the second $CO_2$ separator 20 to the reactor 50. The material gas flow path 60 is provided with a temperature sensor, a flow rate sensor and a $CO_2$ concentration sensor (not shown) configured to measure the temperature, the flow rate and the $CO_2$ concentration of the material gas flowing in the material gas flow path 60.

The heat medium flow path 70 is a flow path which a heat medium of fluid such as oil (thermal fluid) flows in and is configured to supply the heat generated by the methanation reaction in the reactor 50 to the two CO2 separators (first CO2 separator 10 and second CO2 separator 20). The heat medium flow path 70 is provided with a first flow path changeover valve 71 and a second flow path changeover valve 72. The heat medium flow path 70 includes three flow paths (first heat medium flow path 73, second heat medium flow path 74 and third heat medium flow path 75) that are parted from one another by these two valves 71 and 72. The third heat medium flow path 75 is provided with a pump 76 and a temperature controller 77. When the pump 76 is driven, the heat medium in the third heat medium flow path 75 is flowed through the first heat medium flow path 73 or the second heat medium flow path 74 and is then returned to the third heat medium flow path 75 to be circulated. The temperature controller 77 is a device configured to regulate the temperature of the heat medium. When the temperature of the heat medium heated in the reactor 50 is higher than a set temperature, the temperature controller 77 adds a heat medium of ordinary temperature to regulate the temperature of the heat medium. When the temperature of the heat medium is lower than the set temperature, on the other hand, the temperature controller 77 may use a heater or the like to heat the heat medium to the set temperature, along with regulation of the flow rate of the heat medium. The pump 76 and the temperature controller 77 are controlled by the controller 80.

The first flow path changeover valve 71 is a three-way valve configured to change over the destination (the first heat medium flow path 73 or the second heat medium flow path 74) of the heat medium that is delivered from the third heat medium flow path 75 by the pump 76. The second flow path changeover valve 72 is operated in conjunction with the first flow path changeover valve 71 and is a three-way valve configured to change over the source (the first heat medium flow path 73 or the second heat medium flow path 74) of the heat medium that is to be returned to the third heat medium flow path 75. The changeover operations of the first flow path changeover valve 71 and the second flow path changeover valve 72 are controlled by the controller 80.

The third heat medium flow path 75 includes a flow path that is arranged to go through inside of the reactor 50, and is configured to transmit the heat that is generated by the methanation reaction in the reactor 50, to the heat medium. According to the embodiment, the reactor 50 is configured by a double pipe. The catalyst 51 is placed in an inner tube thereof, and a flow path of the heat medium is formed between an outer tube and the inner tube thereof.

The first heat medium flow path 73 includes a flow path that is arranged to go through inside of the first $CO_2$ separator 10, and is configured to supply the heat of the heat medium to the adsorbent 11. The second heat medium flow path 74 includes a flow path that is arranged to go through inside of the second $CO_2$ separator 20, and is configured to supply the heat of the heat medium to the adsorbent 21. Each of the first $CO_2$ separator 10 and the second $CO_2$ separator 20 is also configured by a double pipe and has a flow path of the heat medium that is formed between an outer tube and an inner tube thereof. The first $CO_2$ separator 10 is configured such that the flow direction of the heat medium inside of the first $CO_2$ separator 10 is a direction from a downstream side toward an upstream side of the gas flow direction when $H_2$ is injected from the hydrogen injector 12. According to the embodiment, the flow direction of the heat medium inside of the first $CO_2$ separator 10 is opposite to the injection direction of $H_2$ from the hydrogen injector 12. Similarly, the second $CO_2$ separator 20 is configured such that the flow direction of the heat medium inside of the second $CO_2$ separator 20 is a direction from a downstream side toward an upstream side of the gas flow direction when $H_2$ is injected from the hydrogen injector 22. According to the embodiment, as in the first $CO_2$ separator 10, the flow direction of the heat medium inside of the second $CO_2$ separator 20 is opposite to the injection direction of $H_2$ from the hydrogen injector 22. The heat medium flow path 70 is provided with a temperature sensor and a flow rate sensor (not shown) configured to measure the temperature and the flow rate of the heat medium.

The controller 80 is a computer configured to include a ROM, a RAM and a CPU and serves to control the entire methane production device 1. The controller 80 is electrically connected with the pump 76 and the temperature controller 77 as well as with the valves provided in the respective flow paths described above and the sensors (for example, temperature sensors, flow rate sensors and gas sensors) and controls the valves, the pump 76, the temperature controller 77 and the like, based on measurement values of the sensors and the like. A flow path control process performed by the controller 80 to control the gas flow path and the heat medium flow path will be described later.

The controller 80 estimates an amount $CO_{2\_IN}$ of $CO_2$ flowing into each of the $CO_2$ separators 10 and 20, based on the measurement values of the various sensors (not shown) provided in the exhaust gas supply flow path 30. The controller 80 also estimates an amount $CO_{2\_OUT}$ of $CO_2$ taken out from each of the $CO_2$ separators 10 and 20, based on the measurement values of the various sensors (not shown) provided in the material gas flow path 60. The controller 80 controls the first hydrogen supply valve 42 or the second hydrogen supply valve 43 to adjust an amount $H_{2\_IN}$ of $H_2$ that is to be injected inside of each of the $CO_2$ separators 10 and 20, such that a molar ratio ($H_2/ CO_{2\_IN}$) of $H_2$ and $CO_2$ supplied to the reactor 50 becomes equal to a predetermined value that is not greater than a stoichiometric ratio ($H_2/ CO_{2\_IN}= 4$) in the methanation reaction. The controller 80 also causes the remaining $H_2$ to be supplied from the hydrogen adding portion 45, such that the motor ratio of $H_2$ and $CO_2$ included in the material gas supplied to the reactor 50 becomes equal to 4 ($H_2/ CO_{2\_TOT}= 4$).

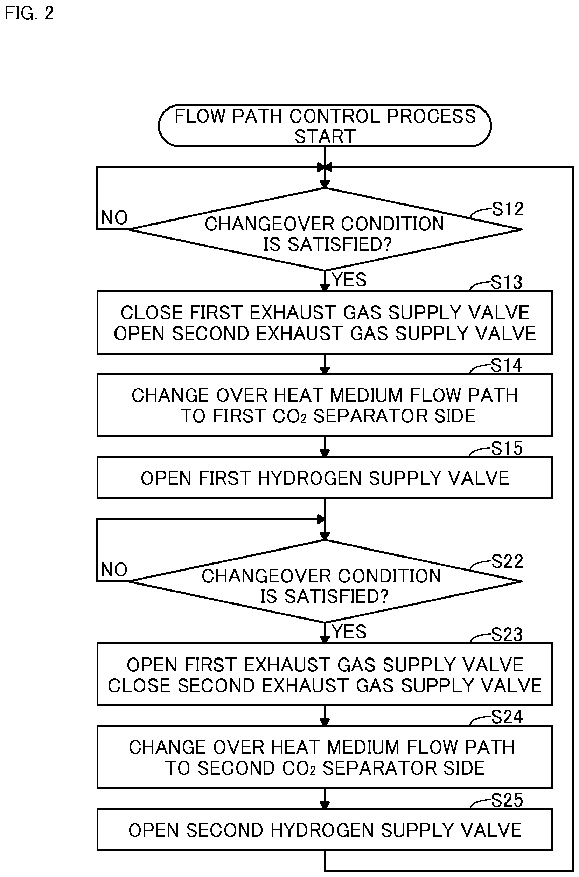

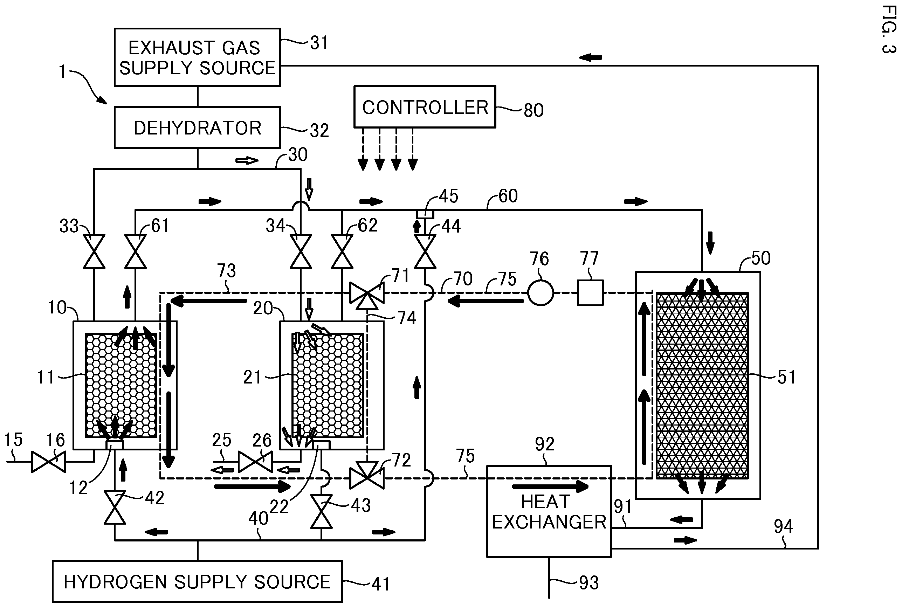

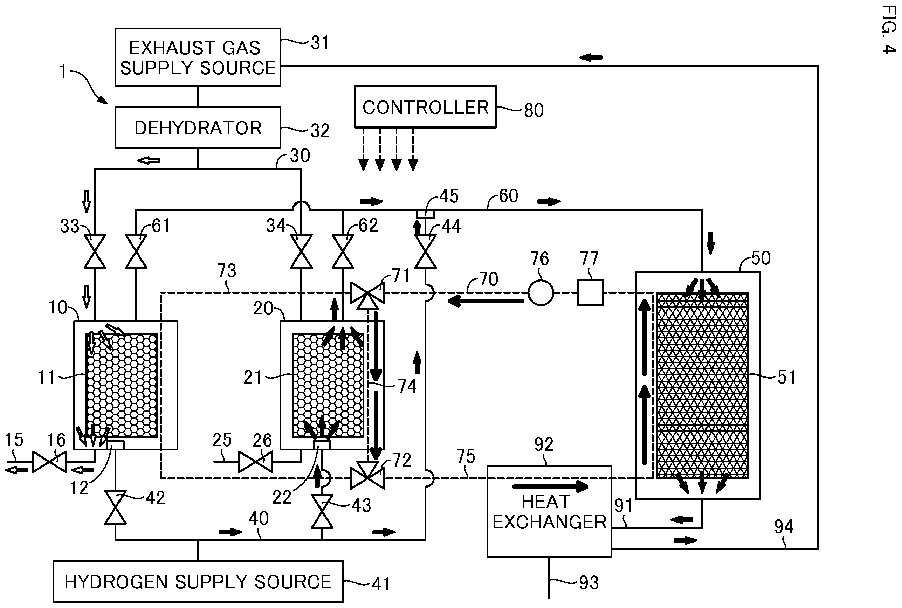

A flow path control process performed by the controller 80 at ordinary time is described below with reference to Figs. 2 to 4. Fig. 2 is a flowchart showing the flow path control process. Fig. 3 is a diagram illustrating the flow state of the gas and the like at steps S13 to S15. Fig. 4 is a diagram illustrating the flow state of gas and the like at steps S23 to S25.

The controller 80 controls the first exhaust gas supply valve 33 to be opened and the second exhaust gas supply valve 34 to be closed at a specific timing during ordinary operation. The controller 80 simultaneously controls the first discharge valve 16 to be opened. Such control causes the dehydrated exhaust gas to be supplied from the exhaust gas supply source 31 to the first $CO_2$ separator 10. $CO_2$ included in the exhaust gas supplied to the first $CO_2$ separator 10 is stored in the adsorbent 11, and the remaining components of the exhaust gas are discharged through the first discharge flow path 15 to the outside.

At this time, the controller 80 determines whether a condition to change over the supply destination of the exhaust gas (changeover condition) is satisfied (step S12). The changeover condition may be any condition that is allowed to estimate that the amount of $CO_2$ stored in the adsorbent 11 becomes equal to a predetermined amount. For example, the controller 80 may determine whether the $CO_2$ concentration in the first discharge flow path 15 becomes higher than a reference value Th1 or may determine whether the amount $CO_{2\_IN}$ of $CO_2$ flowing into the first $CO_2$ separator 10 becomes greater than a reference value Th2. In another example, the controller 80 may determine whether the supply time of the exhaust gas becomes longer than a predetermined time period. The controller 80 keeps supplying the exhaust gas to the first $CO_2$ separator 10 until the changeover condition is satisfied (step S12: NO).

When the changeover condition is satisfied (step S12: YES), the controller 80 controls the first exhaust gas supply valve 33 to be closed and the second exhaust gas supply valve 34 to be opened (step S13). Such control stops the supply of the exhaust gas to the first $CO_2$ separator 10 and starts the supply of the exhaust gas to the second $CO_2$ separator 20. The controller 80 simultaneously controls the second discharge valve 26 to be opened. This causes the dehydrated exhaust gas to be supplied from the exhaust gas supply source 31 to the second $CO_2$ separator 20 and causes $CO_2$ to be stored in the adsorbent 21, as shown in the second $CO_2$ separator 20 of Fig. 3. The other components in the exhaust gas are discharged through the second discharge flow path 25 to the outside. The first $CO_2$ separator 10, on the other hand, stops receiving the supply of the exhaust gas and is in such a state that $CO_2$ is sufficiently stored in the adsorbent 11.

The controller 80 controls the first flow path changeover valve 71 and the second flow path changeover valve 72 to change over the heat medium flow path to the first $CO_2$ separator 10-side and turns the pump 76 ON (step S14). This causes the heat medium to be circulated between the first heat medium flow path 73 and the third heat medium flow path 75, as shown in the heat medium flow path 70 of Fig. 3. At this time, the controller 80 controls the temperature controller 77 such that the temperature of the heat medium flowing through the first $CO_2$ separator 10 becomes equal to a $CO_2$ desorption temperature from the adsorbent 11. At ordinary time, the heat of reaction generated in the reactor 50 is supplied to the heat medium. The controller 80 accordingly causes the heat medium after regulation of the temperature by adding the heat medium of ordinary temperature in the temperature controller 77 to be supplied to the first $CO_2$ separator 10. The heat added by the temperature controller 77 may be supplied to the first $CO_2$ separator 10.

In parallel to turning the pump 76 ON, the controller 80 controls the first hydrogen supply valve 42 to be opened (step S15). Simultaneously, the controller 80 controls the first discharge valve 16 to be closed and the first material gas valve 61 to be opened. Opening the first hydrogen supply valve 42 causes $H_2$ to be injected from the hydrogen injector 12 to the inside of the first $CO_2$ separator 10, as shown in the first $CO_2$ separator 10 of Fig. 3. Injection of $H_2$ causes $CO_2$ in the gas phase desorbed from the adsorbent 11 to be purged out from inside of the first $CO_2$ separator 10 and to be discharged along with the injected $H_2$ to the material gas flow path 60. The controller 80 may control the injection amount $H_{2\_IN}$ of $H_2$ such as to maximize the amount $CO_{2\_OUT}$ by using, for example, the measurement values of the various sensors and a map. In this case, the controller 80 controls the injection amount $H_{2\_IN}$ of $H_2$ such that the injection amount $H_{2\_IN}$ does not become higher than the molar number of the stoichiometric ratio ($H_2/CO_{2\_IN} = 4$) in the methanation reaction. When the injection amount $H_{2\_IN}$ is lower than the stoichiometric ratio, the controller 80 adds the remaining $H_2$ from the hydrogen adding portion 45, such that the material gas to be supplied to the reactor 50 has the stoichiometric ratio in the methanation reaction.

The material gas having a ratio close to the stoichiometric ratio in the methanation reaction is then supplied to the reactor 50. The reactive gaseous mixture including methane generated inside of the reactor 50 is supplied through the reactive gaseous mixture flow path 91 to the heat exchanger 92. $H_2O$ separated in the heat exchanger 92 is discharged through the $H_2O$ discharge flow path 93 to the outside. The reactive gas after separation of $H_2O$ is supplied through the reactive gas flow path 94 to the exhaust gas supply source 31 as fuel gas. At this time, the second $CO_2$ separator 20 keeps receiving the supply of the exhaust gas.

The controller 80 subsequently determines whether a condition to change over the supply destination of the exhaust gas (changeover condition) is satisfied (step S22). The changeover condition may be any condition that is allowed to estimate that the amount of $CO_2$ stored in the adsorbent 21 becomes equal to a predetermined amount. The controller 80 keeps supplying the exhaust gas to the second $CO_2$ separator 20 until the changeover condition is satisfied (step S22: NO).

When the changeover condition is satisfied (step S22: YES), the controller 80 controls the first exhaust gas supply valve 33 to be opened and the second exhaust gas supply valve 34 to be closed (step S23). Such control restarts the supply of the exhaust gas to the first $CO_2$ separator 10 and stops the supply of the exhaust gas to the second $CO_2$ separator 20. The controller 80 simultaneously controls the first discharge valve 16 to be opened. This causes the dehydrated exhaust gas to be supplied from the exhaust gas supply source 31 to the first $CO_2$ separator 10 again and causes $CO_2$ to be stored in the adsorbent 11, as shown in the first $CO_2$ separator 10 of Fig. 4. The second $CO_2$ separator 20, on the other hand, stops receiving the supply of the exhaust gas and is in such a state that $CO_2$ is sufficiently stored in the adsorbent 21.

The controller 80 controls the first flow path changeover valve 71 and the second flow path changeover valve 72 to change over the heat medium flow path to the second $CO_2$ separator 20-side and turns the pump 76 ON (step S24). This causes the heat medium to be circulated between the second heat medium flow path 74 and the third heat medium flow path 75, as shown in the heat medium flow path 70 of Fig. 4. At this time, the controller 80 controls the temperature controller 77 such that the temperature of the heat medium flowing through the second $CO_2$ separator 20 becomes equal to a $CO_2$ desorption temperature from the adsorbent 21, like step S14.

In parallel to turning the pump 76 ON, the controller 80 controls the second hydrogen supply valve 43 to be opened (step S25). Simultaneously, the controller 80 controls the second discharge valve 26 to be closed and the second material gas valve 62 to be opened. Opening the second hydrogen supply valve 43 causes $H_2$ to be injected from the hydrogen injector 22 to the inside of the second $CO_2$ separator 20, as shown in the second $CO_2$ separator 20 of Fig. 4. Injection of $H_2$ causes $CO_2$ in the gas phase desorbed from the adsorbent 21 to be purged out from inside of the second $CO_2$ separator 20 and to be discharged along with the injected $H_2$ to the material gas flow path 60. The controller 80 may control the injection amount $H_{2\_IN}$ of $H_2$, like step S15.

The material gas taken out from the inside of the second $CO_2$ separator 20 is supplied to the reactor 50. The reactive gaseous mixture including methane generated inside of the reactor 50 is supplied through the reactive gaseous mixture flow path 91 to the heat exchanger 92. The reactive gas after separation of $H_2O$ is supplied through the reactive gas flow path 94 to the exhaust gas supply source 31. At this time, the first $CO_2$ separator 10 keeps receiving the supply of the exhaust gas. The controller 80 then sequentially repeats the series of processing starting from step S12. The flow path control process is performed as described above.

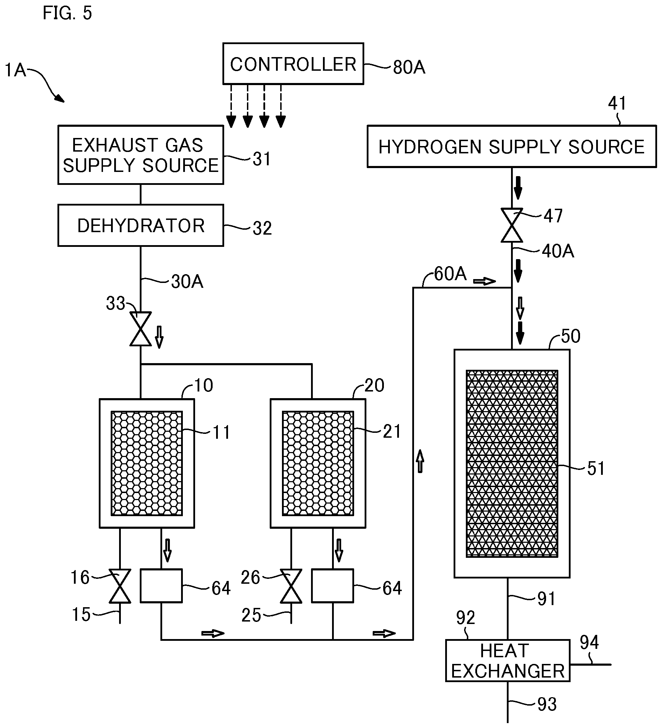

Fig. 5 is a diagram illustrating the schematic configuration of a methane production device 1A according to Comparative Example 1. Exemplary advantageous effects of the embodiment are described, based on comparison between the prior art methane production device and the methane production device of the embodiment. The methane production device 1A of Comparative Example 1 is not provided with the heat medium flow path 70 or the hydrogen injectors 12 and 22, unlike the methane production device 1 of the embodiment (shown in Fig. 1). The methane production device 1A of Comparative Example 1 is, on the other hand, provided with a vacuum pump 64 configured to take out a gas inside of the first $CO_2$ separator 10 to a material gas flow path 60A.

In the methane production device 1A of Comparative Example 1, opening the first exhaust gas supply valve 33 and the first discharge valve 16 causes the dehydrated exhaust gas to be supplied from the exhaust gas supply source 31 to the first $CO_2$ separator 10. $CO_2$ included in the exhaust gas supplied to the first $CO_2$ separator 10 is stored in the adsorbent 11, and the remaining components of the exhaust gas are discharged through the first discharge flow path 15 to the outside. CO2 desorbed from the adsorbent 11 is taken out to the material gas flow path 60A by means of the vacuum pump 64. A hydrogen supply flow path 40A is connected with the material gas flow path 60A. Opening the hydrogen supply valve 47 causes $H_2$ to be supplied from the hydrogen supply source 41 to the material gas flow path 60A. $CO_2$ and $H_2$ flowing in the material gas flow path 60A are supplied to the reactor 50 and are subjected to methanation in the reactor 50.

Unlike the methane production device 1 of the embodiment, the methane production device 1A of Comparative Example 1 is not configured to supply the heat required for desorption of $CO_2$ from the adsorbent 11 through the heat medium flow path 70. This accordingly increases the energy externally applied for the purpose of recycle of $CO_2$ ($CO_2$ recycle energy). The methane production device 1 of the embodiment is, on the other hand, provided with the heat medium flow path 70 and thereby enables the thermal energy generated by the methanation reaction in the reactor 50 to be used as thermal energy required for desorption of $CO_2$ from the adsorbent 11. This reduces the $CO_2$ recycle energy.

As described above, the methane production device 1A of Comparative Example 1 is configured such that $CO_2$ desorbed from the adsorbent 11 is taken out by means of the vacuum pump 64. This further increases the $CO_2$ recycle energy. The methane production device 1 of the embodiment is, on the other hand, configured such that $H_2$ is injected to the inside of the $CO_2$ separator 10 or 20 to purge out $CO_2$. This configuration enhances the recycle rate of $CO_2$ and further reduces the $CO_2$ recycle energy.

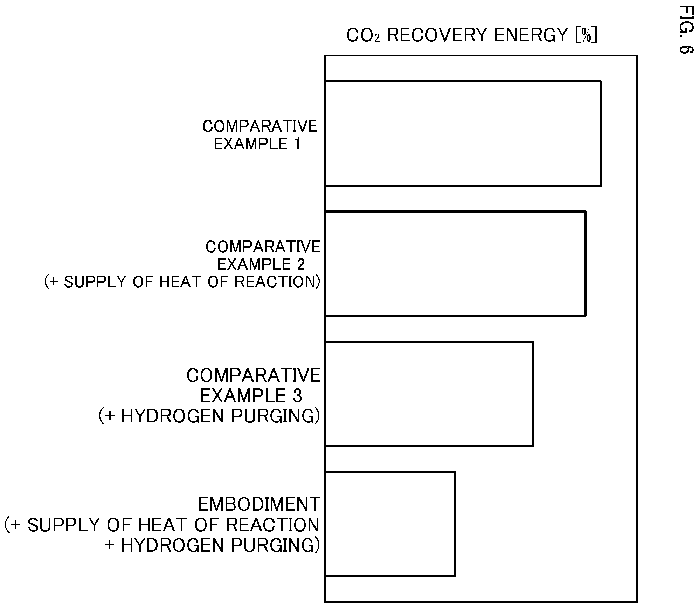

Fig. 6 is a graph showing comparison of $CO_2$ recycle energy between the embodiment and Comparative Examples 1 to 3. More specifically, the graph of Fig. 6 shows $CO_2$ recycle energy of the methane production device 1A of Comparative Example 1 described above, $CO_2$ recycle energy of a methane production device of Comparative Example 2 provided with a configuration of supplying the heat of reaction generated in a reactor to a $CO_2$ separator like the heat medium flow path 70, $CO_2$ recycle energy of a methane production device of Comparative Example 3 provided with a configuration of injecting $H_2$ to a $CO_2$ separator to purge out $CO_2$, and $CO_2$ recycle energy of the methane production device 1 of the embodiment provided with both these configurations. The reduced amount of the $CO_2$ recycle energy of the embodiment relative to the $CO_2$ recycle energy of Comparative Example 1 is greater than the total reduced amount of the $CO_2$ recycle energy of Comparative Examples 2 and 3 relative to the $CO_2$ recycle energy of Comparative Example 1. This shows that the combined use of the configuration of supplying the heat of reaction generated in the reactor to the $CO_2$ separator and the configuration of injecting $H_2$ into the $CO_2$ separator to purge out $CO_2$ like the embodiment enhances the effect of reducing the $CO_2$ recycle energy.

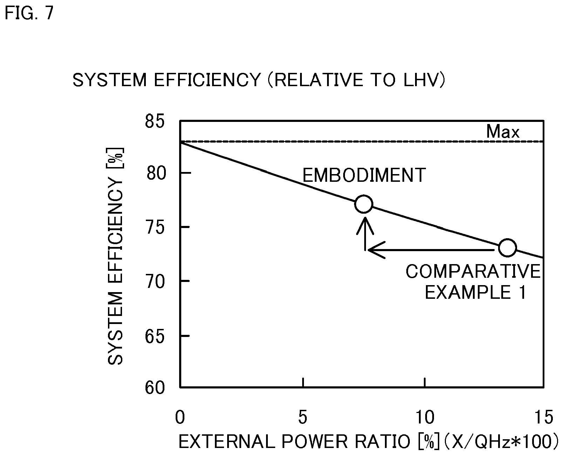

Fig. 7 is a graph showing comparison of system efficiency between the embodiment and Comparative Example 1. The graph of Fig. 7 shows external power ratio [%] as abscissa and system efficiency [%] as ordinate. The external power (electric power) denotes energy externally applied to the methane production device 1 and includes the $CO_2$ recycle energy described above and energies applied to the dehydrator 32 and the heat exchanger 92. The external power ratio denotes a ratio of the external power to a lower heat value (LHV) of hydrogen supplied as the material. The system efficiency denotes an index relating to the energy efficiency of the methane production device. The system efficiency is proportional to a conversion ratio and decreases with an increase in external power. The system efficiency η may be expressed by Expression (1) given below:

$$\eta = 802.3 \times \text{Conv.} / (241.8 \times 4 + X) \tag{1}$$

where Conv. represents the conversion ratio and X represents the external power. The higher system efficiency results in more decreasing the cost of production of methane. The methane production device 1 of the embodiment employs the configuration of heat supply by the heat medium flow path 70 and the configuration of injection of $H_2$ into the $CO_2$ separator and thereby reduces the $CO_2$ recycle energy, compared with the methane production device 1A of Comparative Example 1. This accordingly reduces the external power ratio and enhances the system efficiency, compared with the methane production device 1A of Comparative Example 1.

The methane production device 1 of the embodiment described above is configured to take out $CO_2$ from the $CO_2$ separators 10 and 20 by injecting $H_2$ to the inside of the $CO_2$ separators 10 and 20. This configuration reduces the external power such as a vacuum pump to take out $CO_2$ and enhances the system efficiency of the methane production device. The methane production device 1 of the embodiment is also configured to supply the heat of reaction generated in the reactor 50 to the $CO_2$ separators 10 and 20. This configuration reduces the external power to supply the thermal energy to the $CO_2$ separators 10 and 20 and further enhances the system efficiency of the methane production device. In the methane production device 1 of the embodiment, the $CO_2$ separators 10 and 20 are provided separately from the reactor 50. This configuration enables the heat of reaction generated in the reactor 50 to be supplied to appropriate positions in the $CO_2$ reactors 10 and 20. This enhances the $CO_2$ recycle rate.

The methane production device 1 of the embodiment uses $H_2$ as the purge gas and thereby suppresses reduction of the $CO_2$ recycle rate. More specifically, the selectivity of $CO_2/H_2$ is lower than the selectivity of $CO_2/N_2$ in the adsorbents 11 and 21. This means that $H_2$ used as the purge gas is more unlikely to remain in the adsorbent than $N_2$ used as the purge gas. This accordingly suppresses interference with adsorption of $CO_2$ and suppresses reduction of the $CO_2$ recycle rate. Moreover, $H_2$ is the material used for the methanation reaction. The configuration of the embodiment may thus be implemented by simply moving an inlet of $H_2$ from a gas supply flow path to the reactor 50 to inside of the $CO_2$ separator. This configuration thus easily enhances the system efficiency without using any additional device.

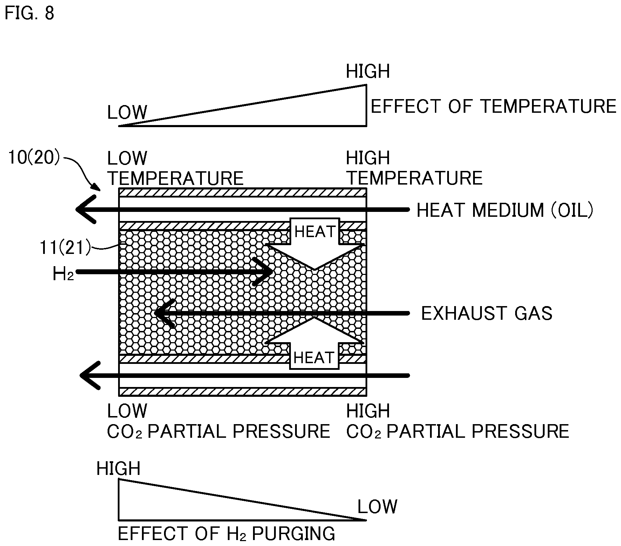

Fig. 8 is a diagram illustrating the internal configuration of the $CO_2$ separator 10 or 20. As described above, the $CO_2$ separator 10 or 20 is configured by a double pipe and has a flow path of the heat medium that is formed between an outer tube and an inner tube thereof. The flow direction of the heat medium is opposite to the flow direction of $H_2$ injected from the hydrogen injector 12 or 22. The supply direction of the exhaust gas is also opposite to the flow direction of $H_2$ injected from the hydrogen injector 12 or 22.

Injection of $H_2$ causes desorbed $CO_2$ to form $H_2/CO_2$ mixed gas, which flows in the injection direction of $H_2$ (purge direction). The arrangement that the flow direction of the heat medium is opposite to the flow direction of the $H_2/CO_2$ mixed gas increases the temperature on the downstream side of the $H_2/CO_2$ mixed gas. This configuration suppresses $CO_2$ included in the $H_2/CO_2$ mixed gas from being adsorbed again into the adsorbent 11 or 21 on the downstream side of the $CO_2$ separator 10 or 20. This enhances the recycle rate of $CO_2$. The $CO_2$ separator 10 or 20 may be configured to change the inlet position of the heat medium. For example, the $CO_2$ separator 10 or 20 may be configured to branch the flow of the heat medium and introduce the branched flows of the heat medium from a plurality of different positions. In another example, the $CO_2$ separator 10 or 20 may be configured to change the inlet position of the heat medium with time. Additionally, the injection direction of $H_2$ is opposite to the supply direction of the exhaust gas, so that $H_2$ may be injected from a lower $CO_2$ partial pressure side toward a higher $CO_2$ partial pressure side. This enables $CO_2$ to be more efficiently taken out from the $CO_2$ separator 10 or 20.

Second Embodiment

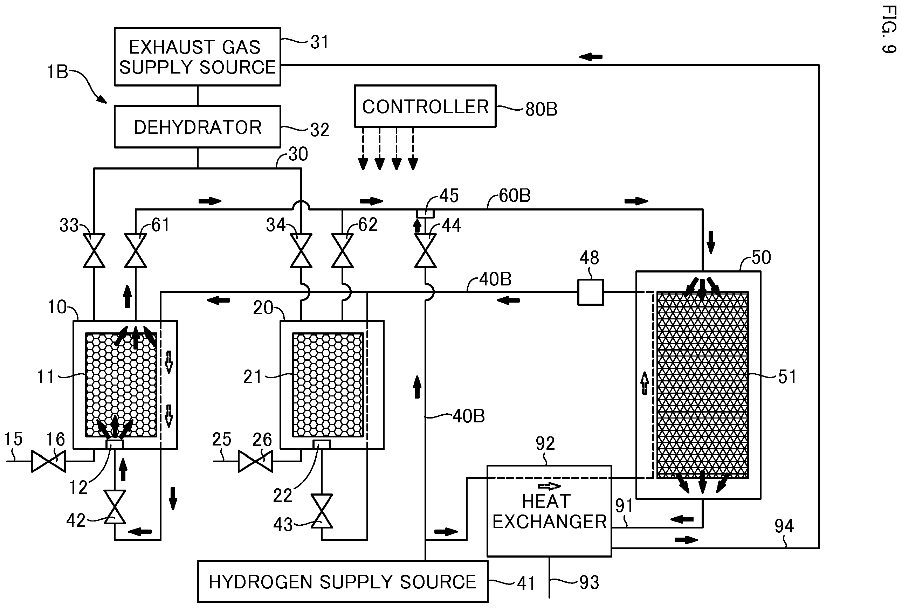

Fig. 9 is a diagram illustrating the schematic configuration of a methane production device 1B according to a second embodiment. Unlike the methane production device 1 of the first embodiment (shown in Fig. 1), the methane production device 1B of the second embodiment is not provided with a heat medium flow path which oil or the like is circulated through (heat medium flow path 70 of the first embodiment). A hydrogen supply flow path 40B of the second embodiment is, on the other hand, configured to use $H_2$ gas that is to be supplied to the $CO_2$ separators 10 and 20 as a heat medium and supply the heat of reaction generated in a reactor 50 to the $CO_2$ separators 10 and 20. In other words, the hydrogen supply flow path 40B also serves as a heat medium flow path according to the second embodiment.

The hydrogen supply flow path 40B includes a flow path that is arranged to go through inside of the reactor 50, and is configured to transmit the heat of reaction generated in the reactor 50 to $H_2$ gas. According to this embodiment, the reactor 50 is configured by a double pipe. A catalyst 51 is placed in an inner tube thereof, and a flow path of $H_2$ gas is formed between an outer tube and the inner tube thereof. The hydrogen supply flow path 40B also includes flow paths that are arranged to go through inside of the two $CO_2$ separators 10 and 20, and is configured to supply the heat of the $H_2$ gas to the adsorbents 11 and 21. Each of the $CO_2$ separators 10 and 20 is also configured by a double pipe and has a flow path of the $H_2$ gas that is formed between an outer tube and an inner tube thereof. The $H_2$ gas flowing in the flow path formed between the outer tube and the inner tube of the $CO_2$ separator 10 or 20 is injected from the hydrogen injector 12 or 122 to the inside of the $CO_2$ separator 10 or 20. The hydrogen supply flow path 40B is further provided with a temperature controller 48. The temperature controller 48 is a device configured to regulate the temperature of the $H_2$ gas that is to be supplied to the $CO_2$ separators 10 and 20, and is controlled by a controller 80B.

When the first hydrogen supply valve 42 is opened, the $H_2$ gas of the hydrogen supply source 41 is heated in the reactor 50, is subjected to temperature regulation by the temperature controller 48, passes through the internal flow path of the first $CO_2$ separator 10 and is injected from the hydrogen injector 12 to the inside of the first $CO_2$ separator 10. The heated $H_2$ gas passes through the internal flow path of the first $CO_2$ separator 10 to increase the temperature of the adsorbent 11 and to cause $CO_2$ to be desorbed from the adsorbent 11. Similarly, when the second hydrogen supply valve 43 is opened, the $H_2$ gas of the hydrogen supply source 41 is heated in the reactor 50, is subjected to temperature regulation by the temperature controller 48, passes through the internal flow path of the second $CO_2$ separator 20 and is injected from the hydrogen injector 22 to the inside of the second $CO_2$ separator 20.

The methane production device 1B of the second embodiment described above is configured to supply the thermal energy generated in the reactor 50 to the $CO_2$ separators 10 and 20 without the heat medium flow path which oil or the like is circulated through. Additionally, the methane production device 1B of the second embodiment does not need to be provided with a circulation device of the heat medium such as oil. This achieves downsizing of the methane production device.

Third Embodiment

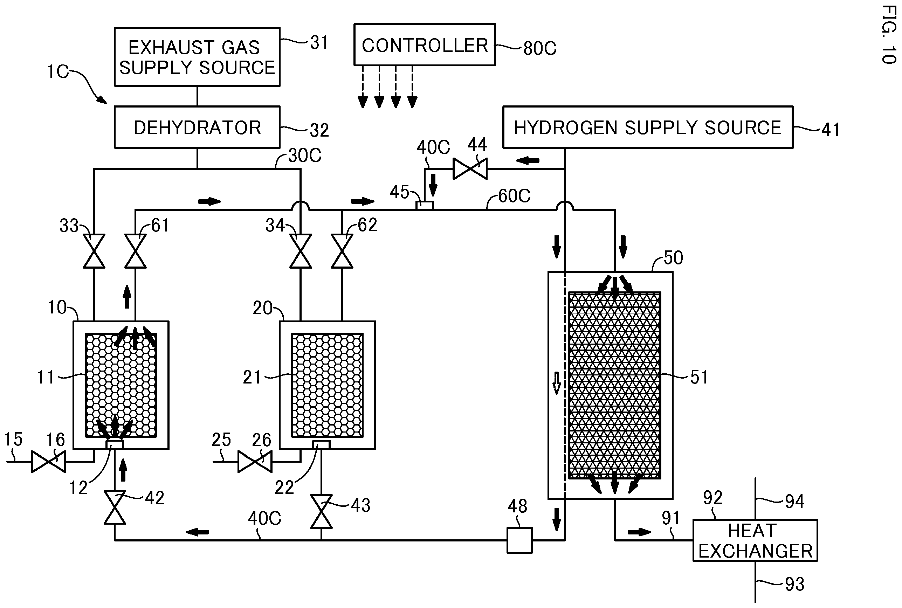

Fig. 10 is a diagram illustrating the schematic configuration of a methane production device 1C according to a third embodiment. Unlike the methane production device 1B of the second embodiment, the methane production device 1C of the third embodiment is not provided with the internal flow path that is formed inside of the $CO_2$ separator 10 or 20 to allow the $H_2$ gas to flow through. A hydrogen supply flow path 40C of the third embodiment is configured such that the $H_2$ gas heated by the heat of reaction generated in a reactor 50 is injected to the inside of the $CO_2$ separator 10 or 20.

When the first hydrogen supply valve 42 is opened, the $H_2$ gas of the hydrogen supply source 41 is heated in the reactor 50, is subjected to temperature regulation by the temperature controller 48 and is injected from the hydrogen injector 12 to the inside of the first $CO_2$ separator 10. Injecting the heated $H_2$ gas increases the temperature of the adsorbent 11 to cause $CO_2$ to be desorbed from the adsorbent 11 and causes the desorbed $CO_2$ to be discharged along with the injected $H_2$ from inside of the $CO_2$ separator 10 to a material gas flow path 60C. Similarly, when the second hydrogen supply valve 43 is opened, the $H_2$ gas of the hydrogen supply source 41 is heated in the reactor 50, is subjected to temperature regulation by the temperature controller 48 and is injected from the hydrogen injector 22 to the inside of the second $CO_2$ separator 20.

The methane production device 1C of the third embodiment described above does not need to be provided with a circulation device of the heat medium such as oil. This achieves downsizing of the methane production device. Moreover, the methane production device 1C of the third embodiment is configured to inject the heated $H_2$ gas and thereby enables the thermal energy to be more efficiently supplied to the adsorbent. The methane production device 1C may be provided with a hydrogen injector configured to inject $H_2$ gas of ordinary temperature, in addition to the hydrogen injectors 12 and 22 configured to inject the heated $H_2$ gas, in the $CO_2$ separators 10 and 20. This configuration enables both the heated $H_2$ gas and the $H_2$ gas of ordinary temperature to be injected to the inside of the $CO_2$ separators 10 and 20 and enables the temperature of the $H_2$ gas to be adjusted to the temperature suitable for desorption of $CO_2$.

Fourth Embodiment

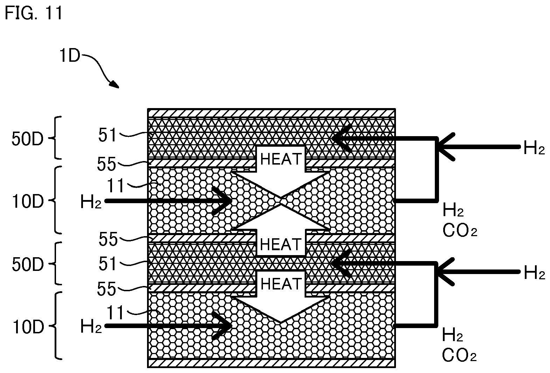

Fig. 11 is a diagram illustrating the schematic configuration of $CO_2$ separators 10D and reactors 50D according to a fourth embodiment. A methane production device 1D of the fourth embodiment is configured such that the $CO_2$ separator 10D and the reactor 50D are adjacent to each other across a partition member 55. More specifically, the methane production device 1D is configured by stacking a plurality of $CO_2$ separators 10D and a plurality of reactors 50D arranged across partition members 55. This configuration enables the heat of reaction generated in the respective reactors 50D to be directly supplied to the $CO_2$ separators 10D via the partition members 55.

The methane production device 1D of the fourth embodiment described above uses the partition members 55 as the heat medium to supply the heat of reaction generated in the reactors 50D, to the $CO_2$ separators 10D. This simple configuration enables the heat of the methanation reaction generated in the reactors to be supplied to the $CO_2$ separators. This achieves downsizing of the methane production device.

Modifications of Embodiments

The present disclosure is not limited to the embodiments described above but may be implemented by various aspects without departing from the scope of the disclosure. Examples of some possible modifications are given below.

Modification 1

In the methane production device 1 of the first embodiment, an amine-based absorbent that is one chemical absorbent may be provided inside of the $CO_2$ separators 10 and 20, in place of the adsorbents 11 and 12. This modified configuration may need the dehydrator 32 of the smaller capacity or may not need the dehydrator 32 at all. In general, chemical absorption increases the desorption energy of $CO_2$, compared with the adsorbent but enhances the system efficiency.

Modification 2

The methane production device 1 of the first embodiment may use a higher hydrocarbon/ alcohol-generating catalyst (for example, Fe-based catalyst or otherwise Cu, Co or the like may be available) that is provided inside of the reactor 50, in place of the catalyst 51. This modification may be configured to employ the higher pressure as the reaction condition than the pressure condition employed for the catalyst having the methanation performance or may be configured to separate a liquid component from the generated gas and recirculate the remaining gas to an inlet of the reactor 50. The separated liquid component is used as a fuel. This modified configuration also enhances the system efficiency.

Modification 3

The methane production device 1 of the first embodiment may be configured to supply the waste heat of the exhaust gas supply source 31 to the $CO_2$ separators 10 and 20. The waste heat may be used to heat the heat medium flowing in the heat medium flow path 70 or may be used to heat the $H_2$ gas supplied to the $CO_2$ separators 10 and 20. The methane production device 1 of the first embodiment may also be configured such that the waste heat of one of the $CO_2$ separators is supplied to the other $CO_2$ separator. Such modified configurations use the waste heat of the exhaust gas supply source 31 or the waste heat of the $CO_2$ separator as the desorption energy of $CO_2$ and thereby further enhance the system efficiency.

Modification 4

The methane production device 1 of the first embodiment uses oil as the heat medium. The heat medium is, however, not limited to the oil but may be any substance that is capable of supplying heat. The heat medium may be, for example, a molten salt or a gas. The methane production device 1 may not be provided with the heat medium flow path 70. Even such modified configurations still enhance the system efficiency as long as $H_2$ is injected by the hydrogen injectors 12 and 22. It is, however, preferable that the methane production device 1 is provided with the heat medium flow path 70.

Modification 5

In the flow path control process of the first embodiment, the timing of increasing the temperature of the adsorbent by the heat medium and the timing of injection of $H_2$ are limited to those of the above embodiment. For example, the temperature of the adsorbent may be increased after injection of $H_2$, or these timings may be shifted arbitrarily.

Modification 6

In the flow path control process of the first embodiment, the temperature controller 77 may serve to heat the heat medium or may not serve to heat the heat medium. For example, the temperature controller 77 may be configured not to heat the heat medium when the heat medium has already been heated by, for example, the heat of reaction generated in the reactor 50.

Modification 7

The methane production device 1 of the first embodiment is provided with the two $CO_2$ separators 10 and 20. The methane production device 1 may, however, be provided with three or more $CO_2$ separators or may be provided

with only one $CO_2$ separator. According to the first embodiment, the first $CO_2$ separator 10 and the second $CO_2$ separator 20 have the identical configurations and identical capacities. According to a modification, the first $CO_2$ separator 10 and the second $CO_2$ separator 20 may have different configurations and different capacities.

Modification 8

The methane production device 1 of the first embodiment may be provided with a vacuum pump configured to take out a gas inside of the first $CO_2$ separator 10 to the material gas flow path 60. Even in this modification, the configuration of the methane production device 1 of the first embodiment reduces the use of the vacuum pump and thereby suppresses an increase in the $CO_2$ recycle energy.

Modification 9

According to the first embodiment, the flow direction of the heat medium inside of the first $CO_2$ separator 10 is opposite to the injection direction of $H_2$ from the hydrogen injector 12. The flow direction of the heat medium inside of the first $CO_2$ separator 10 may, however, not be opposite to the injection direction of $H_2$ from the hydrogen injector 12. It is, however, preferable that the flow direction of the heat medium inside of the first $CO_2$ separator 10 is a direction from the downstream side toward the upstream side of the gas flow direction when $H_2$ is injected from the hydrogen injector 12. It is further preferable that the flow direction of the heat medium inside of the first $CO_2$ separator 10 is opposite to the injection direction of $H_2$ from the hydrogen injector 12.

**Claims**

1. A methane production device (1, 1B, 1C, 1D) configured to produce methane from carbon dioxide and hydrogen, the methane production device comprising:

   a separator (10, 20) provided with an adsorbent (11, 21) that is placed inside thereof and that has a carbon dioxide adsorbing performance and configured to separate carbon dioxide from a gas that is supplied from a supply source of the gas containing carbon dioxide;
   a hydrogen injector (12, 22) configured to inject hydrogen that is supplied from a hydrogen supply source, to inside of the separator (10, 20) and take out carbon dioxide that is desorbed from the adsorbent (11, 21), from the separator (10, 20);
   a reactor (50) provided with a catalyst that is placed inside thereof and that has a methanation performance and configured to cause a methanation reaction by using a material gas including the carbon dioxide taken out from the separator (10, 20) and the hydrogen used in the injection.
   a hydrogen adding portion (45) configured to add hydrogen that is supplied from the hydrogen supply source, to the material gas that is supplied to the reactor (50); and
   a controller (80) configured to control an amount of hydrogen that is to be added by the hydrogen adding portion (45), according to a ratio of the carbon dioxide taken out from the separator (10, 20) to the hydrogen injected from the hydrogen injector (12, 22), and to adjust a ratio of hydrogen and carbon dioxide included in the material gas that is supplied to the reactor (50).

2. The methane production device (1, 1B, 1C, 1D) according to claim 1, further comprising:
   a heat supplier (70, 40B, 40C, 55) configured to supply heat generated in the reactor by the methanation reaction, to the separator (10, 20).

3. The methane production device (1) according to claim 2,

   wherein the heat supplier (70) is configured to cause a heat medium of a fluid to be flowed between the reactor (50) and the separator (10, 20) and thereby to supply the heat generated in the reactor (50) to the separator (10, 20), and
   a flow direction of the heat medium inside of the separator (10, 20) is a direction from a downstream side toward an upstream side of a gas flow direction when hydrogen is injected from the hydrogen injector (12, 22).

4. The methane production device (1B, 1C) according to either claim 2 or claim 3,

   wherein the heat supplier (70) is configured to use hydrogen that is supplied from the hydrogen supply source, as a heat medium to supply the heat generated in the reactor (50) to the separator (10, 20), and
the hydrogen injector (12, 22) is configured to inject hydrogen supplied from the heat supplier (70) to inside of the separator (10, 20).

**5.** The methane production device (1D) according to claim 2,

wherein the reactor (50) and the separator (10, 20) are arranged adjacent to each other, and
the heat supplier (70) uses a member that is provided to part the reactor (50) and the separator (10, 20) from each other, as a heat medium, to supply the heat generated in the reactor (50) to the separator (10, 20).

**6.** A methane production method for producing methane from carbon dioxide and hydrogen, the methane production method comprising:

supplying a gas containing carbon dioxide to a separator provided with an adsorbent that is placed inside thereof and that has a carbon dioxide adsorbing performance, and separating carbon dioxide from the gas;
injecting hydrogen that is supplied from a hydrogen supply source, to inside of the separator and taking out carbon dioxide that is desorbed from the adsorbent, from the separator; and
supplying a material gas including the carbon dioxide taken out from the separator and the hydrogen used in the injection, to a reactor provided with a catalyst that is placed inside thereof and that has a methanation performance, to cause a methanation reaction,
wherein the method further comprises
adding hydrogen that is supplied from the hydrogen supply source, to the material gas that is supplied to the reactor, and
controlling an amount of hydrogen that is to be added to the material gas that is supplied to the reactor, according to a ratio of the carbon dioxide taken out from the separator to the hydrogen injected from the hydrogen injector (12, 22), and adjusting a ratio of hydrogen and carbon dioxide included in the material gas that is supplied to the reactor (50).

## Patentansprüche

**1.** Methanherstellungsvorrichtung (1, 1B, 1C, 1D), die dazu konfiguriert ist, Methan aus Kohlenstoffdioxid und Wasserstoff herzustellen, die aufweist:

einen Separator (10, 20), der mit einem Adsorptionsmittel (11, 21), das darin platziert ist und eine Kohlenstoffdioxid adsorbierende Leistung aufweist, vorgesehen ist und der dazu konfiguriert ist, Kohlenstoffdioxid von einem Gas zu trennen, das von einer Versorgungsquelle für das Kohlenstoffdioxid aufweisende Gas zugeführt wird;
eine Wasserstoffeinspritzvorrichtung (12, 22), die dazu konfiguriert ist, Wasserstoff, der von einer Wasserstoffversorgungsquelle zugeführt wird, in das Innere des Separators (10, 20) einzuspritzen und so Kohlenstoffdioxid, das von dem Adsorptionsmittel (11, 21) desorbiert ist, aus dem Separator (10, 20) zu entnehmen;
einen Reaktor (50), der mit einem Katalysator, der darin platziert ist und eine Methanisierungsleistung aufweist, vorgesehen ist und der dazu konfiguriert ist, eine Methanisierungsreaktion durch Verwendung eines Materialgases, das das aus dem Separator (10, 20) entnommene Kohlenstoffdioxid und den bei dem Einspritzen verwendeten Wasserstoff enthält, zu verursachen;
einen Wasserstoffhinzufügungsabschnitt (45), der dazu konfiguriert ist, Wasserstoff, der von der Wasserstoffversorgungsquelle zugeführt wird, zu dem Materialgas, das an den Reaktor (50) geliefert wird, hinzuzufügen; und
eine Steuereinrichtung (80), die dazu konfiguriert ist, eine Wasserstoffmenge, die durch den Wasserstoffhinzufügungsabschnitt (45) hinzugefügt wird, entsprechend eines Verhältnisses des aus dem Separator (10, 20) entnommenen Kohlenstoffdioxids zu dem von der Wasserstoffeinspritzvorrichtung (12, 22) eingespritzten Wasserstoff, zu steuern und ein Verhältnis von Wasserstoff und Kohlenstoffdioxid, die in dem Materialgas, das an den Reaktor (50) geliefert wird, enthalten sind, einzustellen.

**2.** Methanherstellungsvorrichtung (1, 1B, 1C, 1D) nach Anspruch 1, die weiter aufweist:
einen Wärmelieferanten (70, 40B, 40C, 55), der dazu konfiguriert ist, Wärme, die in dem Reaktor durch die Methanisierungsreaktion erzeugt wird, an den Separator (10, 20) zu liefern.

**3.** Methanherstellungsvorrichtung (1) nach Anspruch 2,

bei der der Wärmelieferant (70) dazu konfiguriert ist, ein Wärmemedium eines Fluids zwischen dem Reaktor (50) und dem Separator (10, 20) strömen zu lassen, und dadurch die in dem Reaktor (50) erzeugte Wärme an den Separator (10, 20) zu liefern, und

eine Strömungsrichtung des Wärmemediums innerhalb des Separators (10, 20) eine Richtung ist, die von stromabwärts in Richtung stromaufwärts einer Gasströmungsrichtung verläuft, wenn Wasserstoff von der Wasserstoffeinspritzvorrichtung (12, 22) eingespritzt wird.

**4.** Methanherstellungsvorrichtung (1B, 1C) nach Anspruch 2 oder 3,

bei der der Wärmelieferant (70) dazu konfiguriert ist, Wasserstoff, der von der Wasserstoffversorgungsquelle zugeführt wird, als ein Wärmemedium zum Liefern der in dem Reaktor (50) erzeugten Wärme an den Separator (10, 20) zu verwenden, und

die Wasserstoffeinspritzvorrichtung (12, 22) dazu konfiguriert ist, Wasserstoff, der von dem Wärmelieferanten (70) zugeführt wird, in den Separator (10, 20) einzuspritzen.

**5.** Methanherstellungsvorrichtung (1D) nach Anspruch 2,

bei der der Reaktor (50) und der Separator (10, 20) benachbart zueinander angeordnet sind, und der Wärmelieferant (70) ein Element, das dafür vorgesehen ist, den Reaktor (50) und den Separator (10, 20) voneinander zu trennen, als ein Wärmemedium verwendet, um die in dem Reaktor (50) erzeugte Wärme an den Separator (10, 20) zu liefern.

**6.** Methanherstellungsverfahren zur Methanherstellung aus Kohlenstoffdioxid und Wasserstoff, das aufweist:

Liefern eines Gases, das Kohlenstoffdioxid enthält, an einen Separator, der mit einem Adsorptionsmittel, das darin platziert ist und eine Kohlenstoffdioxid adsorbierende Leistung aufweist, vorgesehen ist, und Trennen von Kohlenstoffdioxid von dem Gas;

Einspritzen von Wasserstoff, der von einer Wasserstoffversorgungsquelle zugeführt wird, in den Separator und Entnehmen von Kohlenstoffdioxid, das von dem Adsorptionsmittel desorbiert ist, aus dem Separator; und

Liefern eines Materialgases, das den aus dem Separator entnommenen Kohlenstoffdioxid und den bei dem Einspritzen verwendeten Wasserstoff enthält, an einen Reaktor, der mit einem Katalysator, der darin platziert ist und eine Methanisierungsleistung aufweist, vorgesehen ist, um eine Methanisierungsreaktion zu verursachen,

bei dem das Verfahren weiter aufweist:

Hinzufügen von Wasserstoff, der von der Wasserstoffversorgungsquelle zugeführt wird, zu dem Materialgas, das an den Reaktor geliefert wird, und

Steuern einer Menge von Wasserstoff, die dem Materialgas, das an den Reaktor geliefert wird, hinzugefügt wird, entsprechend eines Verhältnisses des aus dem Separator entnommenen Kohlenstoffdioxids zu dem von der Wasserstoffeinspritzvorrichtung (12, 22) eingespritzten Wasserstoff, und Einstellen eines Verhältnisses von Wasserstoff und Kohlenstoffdioxid, die in dem Materialgas, das an den Reaktor (50) geliefert wird, enthalten sind.

**Revendications**

**1.** Dispositif de production de méthane (1, 1B, 1C, 1D) configuré pour produire du méthane à partir de dioxyde de carbone et d'hydrogène, le dispositif de production de méthane comprenant :

un séparateur (10, 20) pourvu d'un adsorbant (11, 21) qui est placé à l'intérieur de celui-ci et qui a une performance d'adsorption du dioxyde de carbone et configuré pour séparer le dioxyde de carbone d'un gaz qui est fourni par une source d'alimentation du gaz contenant du dioxyde de carbone ;

un injecteur d'hydrogène (12, 22) configuré pour injecter de l'hydrogène qui est fourni par une source d'alimentation en hydrogène, à l'intérieur du séparateur (10, 20) et extraire le dioxyde de carbone qui est désorbé de l'adsorbant (11, 21), du séparateur (10, 20) ;

un réacteur (50) muni d'un catalyseur qui est placé à l'intérieur de celui-ci et qui a une performance de méthanation et configuré pour provoquer une réaction de méthanation en utilisant un gaz de matière comprenant le dioxyde de carbone extrait du séparateur (10, 20) et l'hydrogène utilisé dans l'injection,

une partie d'ajout d'hydrogène (45) configurée pour ajouter de l'hydrogène qui est fourni par la source d'alimentation en hydrogène, au gaz de matière qui est fourni au réacteur (50) ; et

un contrôleur (80) configuré pour commander une quantité d'hydrogène qui doit être ajoutée par la partie d'ajout d'hydrogène (45), selon un rapport du dioxyde de carbone extrait du séparateur (10, 20) à l'hydrogène injecté depuis l'injecteur d'hydrogène (12, 22), et pour ajuster un rapport d'hydrogène et de dioxyde de carbone inclus dans le gaz de matière qui est fourni au réacteur (50).

2. Dispositif de production de méthane (1, 1B, 1C, 1D) selon la revendication 1, comprenant en outre :
un fournisseur de chaleur (70, 40B, 40C, 55) configuré pour fournir de la chaleur générée dans le réacteur par la réaction de méthanation, au séparateur (10, 20).

3. Dispositif de production de méthane (1) selon la revendication 2,

dans lequel le fournisseur de chaleur (70) est configuré pour provoquer l'écoulement d'un milieu thermique d'un fluide entre le réacteur (50) et le séparateur (10, 20) et ainsi fournir la chaleur générée dans le réacteur (50) au séparateur (10, 20), et

une direction d'écoulement du milieu thermique à l'intérieur du séparateur (10, 20) est une direction allant d'un côté aval vers un côté amont d'une direction d'écoulement de gaz lorsque l'hydrogène est injecté à partir de l'injecteur d'hydrogène (12, 22).

4. Dispositif de production de méthane (1B, 1C) selon la revendication 2 ou la revendication 3,

dans lequel le fournisseur de chaleur (70) est configuré pour utiliser l'hydrogène qui est fourni par la source d'alimentation en hydrogène, en tant que milieu thermique pour fournir la chaleur générée dans le réacteur (50) au séparateur (10, 20), et

l'injecteur d'hydrogène (12, 22) est configuré pour injecter l'hydrogène fourni par le fournisseur de chaleur (70) à l'intérieur du séparateur (10, 20).

5. Dispositif de production de méthane (1D) selon la revendication 2,

dans lequel le réacteur (50) et le séparateur (10, 20) sont disposés adjacents l'un à l'autre, et

le fournisseur de chaleur (70) utilise un élément qui est prévu pour séparer le réacteur (50) et le séparateur (10, 20) l'un de l'autre, en tant que milieu thermique, pour fournir la chaleur générée dans le réacteur (50) au séparateur (10, 20).

6. Procédé de production de méthane pour produire du méthane à partir de dioxyde de carbone et d'hydrogène, le procédé de production de méthane comprenant les étapes consistant à :

fournir un gaz contenant du dioxyde de carbone à un séparateur muni d'un adsorbant qui est placé à l'intérieur de celui-ci et qui a une performance d'adsorption du dioxyde de carbone, et séparer le dioxyde de carbone du gaz ;

injecter de l'hydrogène qui est fourni à partir d'une source d'alimentation en hydrogène, à l'intérieur du séparateur et retirer le dioxyde de carbone qui est désorbé de l'adsorbant, du séparateur ; et

fournir un gaz de matière comprenant le dioxyde de carbone extrait du séparateur et l'hydrogène utilisé dans l'injection, à un réacteur muni d'un catalyseur qui est placé à l'intérieur de celui-ci et qui a une performance de méthanation, pour provoquer une réaction de méthanation,

dans lequel le procédé comprend en outre les étapes consistant à

ajouter l'hydrogène qui est fourni par la source d'alimentation en hydrogène, au gaz de matière qui est fourni au réacteur, et

contrôler une quantité d'hydrogène qui doit être ajoutée au gaz de matière qui est fourni au réacteur, selon un rapport du dioxyde de carbone extrait du séparateur sur l'hydrogène injecté depuis l'injecteur d'hydrogène (12, 22), et ajuster un rapport d'hydrogène et de dioxyde de carbone inclus dans le gaz de matière qui est fourni au réacteur (50).

FIG. 1

FIG. 2

```
┌─────────────────────────────────┐
│  FLOW PATH CONTROL PROCESS      │
│           START                 │
└─────────────────────────────────┘
                 │
        ┌────────────────────────────────────────┐
        │         ◇ CHANGEOVER CONDITION    S12   │
   NO ──┤           IS SATISFIED?                 │
        │              │ YES              S13      │
        │   ┌──────────────────────────────────┐  │
        │   │ CLOSE FIRST EXHAUST GAS SUPPLY   │  │
        │   │ VALVE OPEN SECOND EXHAUST GAS    │  │
        │   │ SUPPLY VALVE                     │  │
        │   └──────────────────────────────────┘  │
        │              │                  S14      │
        │   ┌──────────────────────────────────┐  │
        │   │ CHANGE OVER HEAT MEDIUM FLOW     │  │
        │   │ PATH TO FIRST CO2 SEPARATOR SIDE │  │
        │   └──────────────────────────────────┘  │
        │              │                  S15      │
        │   ┌──────────────────────────────────┐  │
        │   │ OPEN FIRST HYDROGEN SUPPLY VALVE │  │
        │   └──────────────────────────────────┘  │
        │              │                           │
        │         ◇ CHANGEOVER CONDITION    S22   │
   NO ──┤           IS SATISFIED?                 │
        │              │ YES              S23      │
        │   ┌──────────────────────────────────┐  │
        │   │ OPEN FIRST EXHAUST GAS SUPPLY    │  │
        │   │ VALVE CLOSE SECOND EXHAUST GAS   │  │
        │   │ SUPPLY VALVE                     │  │
        │   └──────────────────────────────────┘  │
        │              │                  S24      │
        │   ┌──────────────────────────────────┐  │
        │   │ CHANGE OVER HEAT MEDIUM FLOW     │  │
        │   │ PATH TO SECOND CO2 SEPARATOR SIDE│  │
        │   └──────────────────────────────────┘  │
        │              │                  S25      │
        │   ┌──────────────────────────────────┐  │
        │   │ OPEN SECOND HYDROGEN SUPPLY VALVE│  │
        │   └──────────────────────────────────┘  │
        └─────────────────────────────────────────┘
```

FIG. 3

FIG. 4

FIG. 5

FIG. 7

SYSTEM EFFICIENCY (RELATIVE TO LHV)

FIG. 8

FIG. 9

EXHAUST GAS SUPPLY SOURCE 31

DEHYDRATOR 32

CONTROLLER 80B

30

1B

33

61

34

62

45 44

60B

48

40B

40B

50

51

94

HEAT EXCHANGER 91

92

93

41

HYDROGEN SUPPLY SOURCE

20 21

25 26

22 43

10 11

12 42

15 16

FIG. 10

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016076041 A
- WO 2011108546 A
- JP 2009269983 A
- JP 5959036 B

- JP H1146460 A
- JP 2009077457 A
- DE 102013022021 A
- CN 104152197 A

**Non-patent literature cited in the description**

- **GÖKHAB ALPETKIN et al.** An Advanced CO2 Removal and Reduction System. *SAE TECHNICAL PAPER SERIES,* 2004, ISSN 0148-7191